(19) Europäisches Patentamt
European Patent Office
Office européen des brevets

(11) EP 1 856 033 B1

(12) **EUROPÄISCHE PATENTSCHRIFT**

(45) Veröffentlichungstag und Bekanntmachung des Hinweises auf die Patenterteilung:
**22.10.2008 Patentblatt 2008/43**

(21) Anmeldenummer: **06707269.4**

(22) Anmeldetag: **24.02.2006**

(51) Int Cl.:
***C07C 253/30*** (2006.01) ***C07C 255/33*** (2006.01)
***C11D 3/39*** (2006.01)

(86) Internationale Anmeldenummer:
**PCT/EP2006/001733**

(87) Internationale Veröffentlichungsnummer:
**WO 2006/092245 (08.09.2006 Gazette 2006/36)**

(54) **VERBESSERTES VERFAHREN ZUR HERSTELLUNG VON BIFUNKTIONELLEN AMMONIUMNITRILEN**

IMPROVED METHOD FOR PRODUCING BI-FUNCTIONAL AMMONIUM NITRILES

PROCEDE AMELIORE POUR PRODUIRE DES NITRILES D'AMMONIUM BIFONCTIONNELS

(84) Benannte Vertragsstaaten:
**BE CH DE ES FR GB IT LI**

(30) Priorität: **01.03.2005 DE 102005009136**

(43) Veröffentlichungstag der Anmeldung:
**21.11.2007 Patentblatt 2007/47**

(73) Patentinhaber: **Clariant Produkte (Deutschland) GmbH**
**65929 Frankfurt am Main (DE)**

(72) Erfinder:
- **CUYPERS, Lars**
  **65835 Liederbach (DE)**
- **REINHARDT, Gerd**
  **65779 Kelkheim (DE)**
- **PREHLER, Hans**
  **65830 Kriftel (DE)**

(74) Vertreter: **Mikulecky, Klaus et al**
**Clariant Produkte (Deutschland) GmbH**
**Group Intellectual Property**
**Am Unisys-Park 1**
**65843 Sulzbach (DE)**

(56) Entgegenhaltungen:
**EP-A- 0 303 520**

- **DATABASE CA [Online] CHEMICAL ABSTRACTS SERVICE, COLUMBUS, OHIO, US; 8. Dezember 1990 (1990-12-08), AOYANAGI, MUNEO ET AL: "Peroxide bleaching agents for detergents" XP002388803 gefunden im STN Database accession no. 113:214317 & JP 02 132196 A2 (KAO CORP., JAPAN) 21. Mai 1990 (1990-05-21)**
- **PATENT ABSTRACTS OF JAPAN Bd. 013, Nr. 501 (C-652), 10. November 1989 (1989-11-10) -& JP 01 198700 A (KAO CORP), 10. August 1989 (1989-08-10)**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 856 033 B1

Printed by Jouve, 75001 PARIS (FR)

**Beschreibung**

Die vorliegende Erfindung betrifft eine verbesserte Synthese von bifunktionellen Ammoniumnitrilen durch einstufige Umsetzung von tertiären Diaminen mit Quaternierungsmitteln.

In der Patentliteratur werden Ammoniumnitrile und deren Verwendung als Bleichaktivatoren in Wasch- und Reinigungsmitteln beschrieben: Als Beispiele seien EP-A-303 520, EP-A-458 396, EP-A-464 880 und WO-A-2 003 078 561 aufgeführt. Durch Zusatz dieser Aktivatoren kann die Bleichwirkung wässriger Peroxidlösungen soweit gesteigert werden, dass bei 40°C im wesentlichen die gleiche Wirkung eintritt, wie sie sonst nur mit der Peroxidlösung allein bei 95°C erzielt wird.

In EP-A-303 520 wird unter anderem die Synthese der bifunktionellen Ammoniumnitrile (I) und (II) beschrieben:

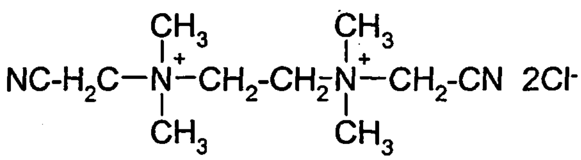

(I)

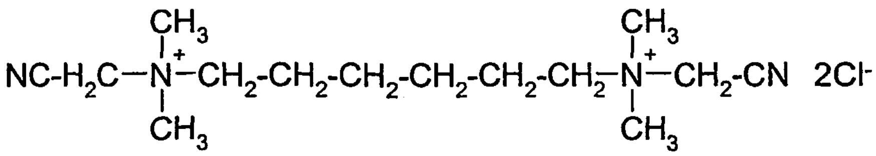

(II)

Gemäß EP-A-303 520 werden diese Ammoniumnitrile durch Umsetzung von Chloracetonitril mit einem Überschuss N,N,N',N'-Tetramethylethylendiamin bzw. N,N,N',N"-Tetramethylhexan-1,6-diamin in Aceton bei 90°C erhalten. Wurden die beschriebenen Synthesen für (I) und (II) nachgestellt, konnten nur unbefriedigende Ausbeuten erhalten werden. Beispielsweise wurde Verbindung (I) nur mit einer Ausbeute von 11 % isoliert.

Es bestand daher die Aufgabe, ein verbessertes Verfahren zur Herstellung von bifunktionellen Ammoniumnitrilen zu finden.

Überraschenderweise wurde gefunden, dass durch Umsetzung von tertiären Diaminen
mit Cyanomethylengruppen übertragenden Quaternierungsmitteln, bifunktionelle Ammoniumnitrile in sehr reiner Form und in hohen Ausbeuten erhalten werden.

Gegenstand der vorliegenden Erfindung ist somit ein Verfahren zur Synthese von Verbindungen der allgemeinen Formel

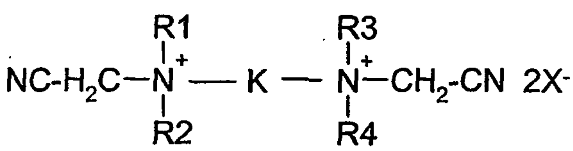

wobei K für eine Gruppe der Formeln

$$-(CH_2)_n-, -(CH_2)_n-A-(CH_2)_n-$$

steht,

A für Sauerstoff bzw. eine Gruppe der Formel $N-R^5$ steht,

$R^1$, $R^2$, $R^3$ und $R^4$ jeweils unabhängig voneinander eine gerad- oder verzweigtkettige $C_1$- bis $C_{24}$-Alkyl-, Alkenyl- oder Alkylethergruppe, vorzugsweise eine $C_1$- bis $C_{18}$-Alkyl-, Alkenyl- oder Alkylethergruppe bedeuten, oder

$R^1$, $R^3$ und K zusammen mit den beiden N-Atomen entweder eine Gruppe der Formel

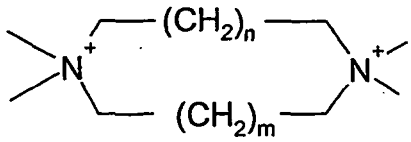

oder $R^1$, $R^2$, $R^3$, $R^4$ und K zusammen mit den beiden N-Atomen eine Gruppe der Formel

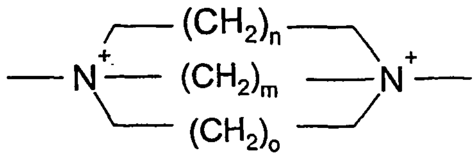

bedeutet,
$R^5$ für Wasserstoff oder eine $C_1$- bis $C_{24}$-Alkyl-, Alkenyl oder Cycloalkylgruppe, vorzugsweise eine $C_1$- bis $C_{18}$-Alkyl-, Alkenyl oder Cycloalkylgruppe steht,
X- ein Anion, beispielsweise Chlorid, Bromid, Jodid, Toluolsulfonat, Benzolsulfonat, Cumolsulfonat oder Mesitylsulfonat ist und
die Variablen m, n und o für ganze Zahlen von 1 bis 16 stehen.

Dieses Verfahren besteht darin, dass man ein tertiäres Diamin der Formel

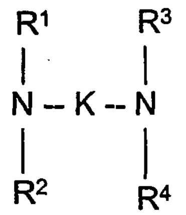

mit einem Cyanomethylengruppen übertragenden Quaternisierungsmittel der allgemeinen Formel

$X-CH_2CN$

in einem polaren, aprotischen Lösungsmittel mit einem Siedepunkt oberhalb 60°C umsetzt, wobei A, $R^1$, $R^2$, $R^3$, $R^4$, $R^5$, X, m, n und o die zuvor angegebenen Bedeutungen haben.

Für das erfindungsgemäße Verfahren geht man im Einzelnen so vor, dass man zunächst das Cyanomethylengruppen übertragende Quaternierungsmittel in einem geeigneten polar-aprotischen Lösungsmittel löst oder suspendiert. Das Lösungsmittel soll einen Siedepunkt oberhalb 60°C aufweisen. Als Lösungsmittel kommen beispielsweise in Frage: Essigsäureethylester, Essigsäure-n-propylester, Essigsäure-i-propylester, Essigsäure-n-butylester, Essigsäure-i-butylester und deren Gemische, Dimethylsulfoxid, N-Methylpyrrolidon, 1,3-Dimethylimidazolidin-2-on. Bevorzugt sind Essigsäureethylester, Dimethylformamid und Dimethylacetamid, besonders bevorzugt ist Dimethylacetamid, da die Ausgangsprodukte in diesem Lösungsmittel eine sehr gute Löslichkeit aufweisen, während die Endprodukte schwer löslich sind und ausfallen. Zu dieser Lösung bzw. Suspension wird ein tertiäres Diamin (III) zugetropft. Die Menge an Quaternierungsmittel beträgt 1,8 bis 3,0, bevorzugt 2,0 bis 2,5, insbesondere 2,0 bis 2,3 Moläquivalente bezogen das tertiäre Diamin (III). Als Quaternierungsmittel kommen in Frage Halogenacetonitrile und Arylacetonitrile, bevorzugt sind Chloracetonitril, Bromacetonitril, Jodacetonitil, Tosylacetonitril und Cumolacetonitril, besonders bevorzugt ist Chloracetonitril. Die Reaktionstemperatur beträgt im Allgemeinen 20 bis 120°C, bevorzugt 30 bis 100°C, besonders bevorzugt 40 bis 80°C. Die Reaktion läuft in einem Zeitraum von 1 bis 10 Stunden, bevorzugt 2 bis 9 Stunden, besonders bevorzugt 3 bis 8 Stunden. Das entstandene Produkt kann durch Filtration, Abnutschen, Abdekantieren oder durch Zentrifugation vom Lösungsmittel abgetrennt werden.

Nachfolgende Beispiele sollen die Erfindung näher erläutern ohne sie darauf einzuschränken.

Beispiel 1: Synthese von N,N,N',N'-Tetramethyl-N,N'-di(cyanomethyl)-1,2-ethandiammoniumdichlorid

37,75 g (0,5 mol) Chloracetonitril wurden in 100 ml Essigsäureethylester vorgelegt und unter Rühren bei Raumtemperatur 29 g (0,25 mol) N,N,N',N'-Tetramethylethylendiamin zugetropft. Das Reaktionsgemisch wurde 5 Stunden bei 50°C gerührt und dann auf Raumtemperatur abgekühlt. Der ausgefallene Feststoff wurde abfiltriert und dreimal mit je 50 ml Essigsäureethylester gewaschen. Der Filterkuchen wurde im Vakuum bei 60°C getrocknet. Man erhielt 60,9 g (0,23 mol) N,N,N',N'-Tetramethyl-N,N'-di(cyanomethyl)-1,2-ethandiammoniumdichlorid als weißen Feststoff, entsprechend einer Ausbeute von 91 %.
Fp.: 183°C (Zersetzung)
Elementaranalyse:
Gef.: C 44,8 %; H 7,5 %; N 21,2 %;Cl 26,5 %
Ber.: C 45,0 %; H 7,5 %; N 21,0 %; Cl 26,5 %
$^1$H-NMR ($D_2O$):
$\delta$ = 4,75 (4 H, s); $\delta$ = 4,34 (4 H, s); $\delta$ = 3,54 (12 H, s)
IR (KBr):
3040 vs, 3020 vs, 2960 vs, 1475 vs, 1440 s, 1405 m, 1350 w, 1300 w, 1250 vw, 1215 vw, 1130 w, 1010 w, 985 vs, 920 vs , 910 s, 795 s, 755 vw

Beispiel 2: Synthese von N,N,N',N'-Tetramethyl-N,N'-di(cyanomethyl)-3-propan-diammoniumdichlorid

57,38 g (0,76 mol) Chloracetonitril wurden in 160 ml N,N-Dimethylacetamid vorgelegt und unter Rühren bei Raumtemperatur 50 g (0,38 mol) N,N,N',N'-Tetramethyl-1,3-propandiamin zugetropft. Das Reaktionsgemisch wurde 5 Stunden bei 50°C gerührt und anschließend auf Raumtemperatur abgekühlt. Der ausgefallene Feststoff wurde abfiltriert und mit N,N-Dimethylacetamid gewaschen. Der Filterkuchen wurde bei 60°C im Vakuum getrocknet. Man erhielt 100,3 g (0,36 mol) N,N,N',N'-Tetramethyl-N,N'-di(cyanomethyl)-1,3-propandiammonium-dichlorid als weißen Feststoff, entsprechend einer Ausbeute von 94 %.
Fp.: 153°C
$^1$H-NMR ($D_2O$):
$\delta$ = 4,75 (4 H, s); $\delta$ = 3,74 (4 H, t); $\delta$ = 3,44 (12 H, s); $\delta$ = 2,54 (2 H, m)

Beispiel 3: Synthese von N,N,N',N'-Tetraethyl-N,N'-di(cyanomethyl)-1,3-propandiammoniumdichlorid

19,6 g (0,26 mol) Chloracetonitril wurden in 75ml N,N-Dimethylacetamid vorgelegt und unter Rühren bei Raumtemperatur 25 g (0,13 mol) Tetraethyl-1,3-propandiamin zugetropft. Das Reaktionsgemisch wurde 7 Stunden bei 55°C gerührt und anschließend auf Raumtemperatur abgekühlt. Der ausgefallene Feststoff wurde abfiltriert und mit N,N-Dimethylacetamid gewaschen. Der Filterkuchen wurde bei 60°C im Vakuum getrocknet. Man erhielt 18,3 g (0,05 mol) N,N,N',N'-Tetraethyl-N,N'-di(cyanomethyl)-1,3-propandiammoniumdichlorid als weißen Feststoff, entsprechend einer Ausbeute von 42 %.
Fp.: 203°C (Zersetzung)
$^1$H-NMR ($D_2O$):
$\delta$ = 4,75 (4 H, s); ö = 3,72 - 3,65 (12 H, m); ö = 2,38 (2H, m); ö = 1,44 (12 H, t)

Beispiel 4: Bleichleistung von bifunktionellen Ammoniumnitrilen

Die Bleichleistung der Cyanomethylammonium-Salze wurde in einem Linitest-Gerät (Fa. Heraus) bei 20, 40 und 60°C untersucht. Hierzu wurden 2 g/l eines bleichmittelfreien Grundwaschmittels (WMP, WFK, Krefeld) und 0,5 g/l Natriumperborat Monohydrat (Fa. Degussa) in Wasser der Härtestufe 3 gelöst. Anschließend wurden entweder 100mg/l, 200 mg/l oder 250 mg/l Aktivator zugegeben. Die Waschzeit betrug 30 min. Anschließend wurden die Gewebestücke mit Wasser gespült, getrocknet und gebügelt. Als Bleichtestgewebe dienten Tee BC-1 und Curry BC-4 (WFK Testgewebe GmbH, Krefeld) auf Baumwolle. Als Bleichergebnis wurde die Remissionsdifferenz, gemessen mit einem Elrepho-Gerät, nach der Wäsche im Vergleich zum Gewebe, gewaschen mit 2 g/l WMP und 0,5 g/l Natriumperborat-Monohydrat, gewertet.

$$\Delta\Delta R = \Delta R(\text{Formulierung+Persalz+Aktivator}) - \Delta R(\text{Formulierung*Persalz})$$

Es wurden Bleichmittelzusammensetzungen mit den erfindungsgemäßen kationischen Nitrilverbindungen 1, 2 und 3 sowie der Vergleichssubstanzen 4 hergestellt und getestet.

Die Verbindungen 1 bis 4 sind

1 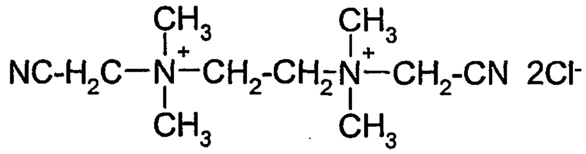

2 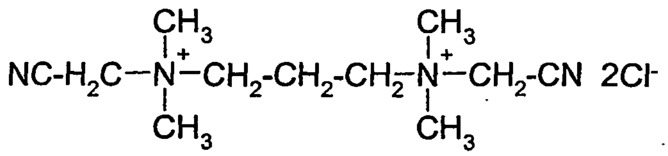

3 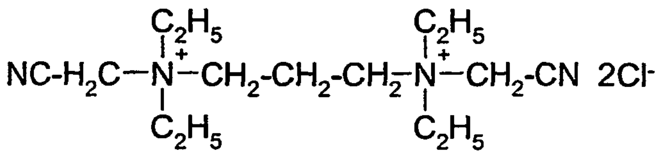

4 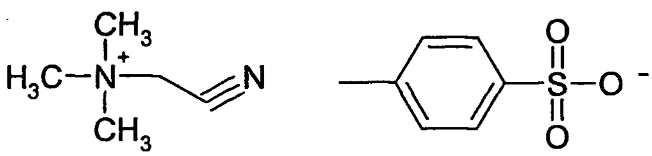

Mit den Aktivatoren 1 und 3 wurden Waschversuche an den Bleichtestgeweben. Tee BC-1 und Curry BC-4 bei 20, 40 und 60°C bei Konzentrationen von 0,1 bzw. 0,2 g/l durchgeführt. Die Ergebnisse sind in Tabelle 1 dargestellt:

Tabelle 1: Prüfergebnisse (ΔΔR-Werte) für die Aktivatoren 1 und 3 an Tee BC-1 und Curry BC-4

| Waschbedingungen | Aktivator 1 | Aktivator 3 | Aktivator 1 | Aktivator 3 |
|---|---|---|---|---|
| | Tee BC-1 | | Curry BC-4 | |
| 20°C; c(Aktivator) = 0,1 g/l | 9,6 | 6,9 | 3,8 | 2,9 |
| 20°C; c(Aktivator) = 0,2 g/l | 14,2 | 9,4 | 4,0 | 3,9 |
| 40°C; c(Aktivator) = 0,1 g/l | 8,5 | 10,2 | 2,9 | 4,3 |
| 40°C; c(Aktivator) = 0,2 g/l | 13,9 | 10,3 | 3,2 | 4,1 |
| 60°C; c(Aktivator) = 0,1 g/l | 5,3 | 2,8 | 2,2 | 1,2 |
| 60°C; c(Aktivator) = 0,2 g/l | 7,1 | 5,7 | 1,8 | -0,4 |

Für die Aktivatoren 1 und 2 wurden Waschversuche bei 20 bzw. 40°C bei Konzentrationen von 0,1 bzw, 0,2

g/l durchgeführt. Die Ergebnisse sind in Tabelle 2 dargestellt:

Tabelle 2: Prüfergebnisse (ΔΔR-Werte) für die Aktivatoren 1 und 2 an Tee BC-1 und Curry BC-4

| Waschbedingungen | Aktivator 1 | Aktivator 2 | Aktivator 1 | Aktivator 2 |
|---|---|---|---|---|
| | Tee BC-1 | | Curry BC-4 | |
| 20°C; c(Aktivator) = 0,1 g/l | 10,6 | 9,8 | 4,1 | 4,7 |
| 20°C; c(Aktivator) = 0,2 g/l | 14,9 | 14,5 | 5,4 | 5,7 |
| 40°C; c(Aktivator) = 0,1 1g/l | 11,5 | 12,5 | 3,4 | 4,1 |
| 40°C; c(Aktivator) = 0,2 g/l | 14,3 | 16,5 | 3,5 | 4,4 |

Des Weiteren wurden mit Aktivator 1 Waschversuche bei 20 bzw. 40°C bei einer Konzentration von 0,25 g/l durchgeführt. Die Ergebnisse werden in Tabelle 3 mit dem hydrophoben Aktivator 4 verglichen:

Tabelle 3: Prüfergebnisse (ΔΔR-Werte) für die Aktivatoren 1 und 4 bei 20 und 40°C an diversen Testanschmutzungen

| | Aktivator 1 | Aktivator 4 | Aktivator 1 | Aktivator 4 |
|---|---|---|---|---|
| | 20°C | | 40°C | |
| Schwarze Johannisbeere | 1,7 | 1,8 | 0,9 | 0,6 |
| Chlorophyll | 3,0 | 0,1 | 2,0 | 1,1 |
| Rotwein CS-3 | 13,6 | 4,6 | 10,8 | 5,6 |
| Gras | 9,4 | 2,3 | 8,4 | 2,2 |
| Ketchup | 3,0 | 0,6 | 1,3 | 1,0 |
| Tee BC-3 | 25,3 | 8,4 | 23,6 | 12,5 |

**Patentansprüche**

1. Verfahren zur Herstellung von bifunktionellen Ammoniumnitrilen der Formel

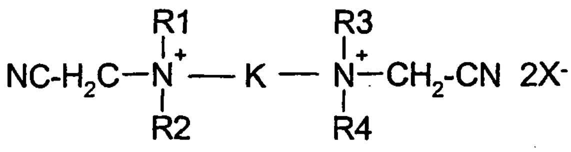

wobei K für eine Gruppe der Formeln

$$-(CH_2)_n-, -(CH_2)_n-A-(CH_2)_n-$$

steht,
A für Sauerstoff bzw. eine Gruppe der Formel $N-R^5$ steht,
$R^1$, $R^2$, $R^3$ und $R^4$ jeweils unabhängig voneinander eine gerad- oder verzweigtkettige $C_1$- bis $C_{24}$-Alkyl-, Alkenyl- oder Alkylethergruppe, vorzugsweise eine $C_1$- bis $C_{18}$-Alkyl-, Alkenyl- oder Alkylethergruppe bedeuten, oder
$R^1$, $R^3$ und K zusammen mit den beiden N-Atomen entweder eine Gruppe der Formel

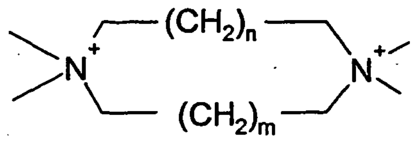

oder $R^1$, $R^2$, $R^3$, $R^4$ und K zusammen mit den beiden N-Atomen eine Gruppe der Formel

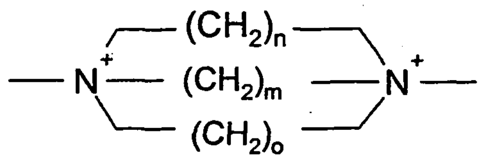

bedeutet,
$R^5$ für Wasserstoff oder eine $C_1$- bis $C_{24}$-Alkyl-, Alkenyl oder Cycloalkylgruppe, vorzugsweise eine $C_1$- bis $C_{18}$-Alkyl-, Alkenyl oder Cycloalkylgruppe steht,
X- ein Anion, beispielsweise Chlorid, Bromid, Jodid, Toluolsulfonat, Benzolsulfonat, Cumolsulfonat oder Mesitylsulfonat ist und
die Variablen m, n und o für ganze Zahlen von 1 bis 16 stehen, durch Umsetzung eines tertiären Diamins der Formel

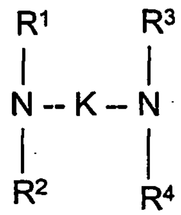

mit einem Cyanomethylengruppen übertragenden Quaternisierungsmittel der allgemeinen Formel

$X\text{-}CH_2CN$

**dadurch gekennzeichnet, daß** man die Umsetzung in einem polaren, aprotischen Lösungsmittel mit einem Siedepunkt oberhalb 60°C durchführt, wobei A, $R^1$, $R^2$, $R^3$, $R^4$, $R^5$, X, m, n und o die zuvor angegebenen Bedeutungen haben.

2. Verfahren nach Anspruch 1, **dadurch gekennzeichnet, dass** als Quaternierungsmittel der Formel $X\text{-}CH_2CN$ Halogenacetonitrile und Arylacetonitrile verwendet werden.

3. Verfahren nach Anspruch 1, **dadurch gekennzeichnet, dass** als Quaternierungsmittel der Formel $X\text{-}CH_2CN$ Chloracetonitril, Bromacetonitril, Jodacetonitil, Tosylacetonitril und Cumolacetonitril verwendet werden:

4. Verfahren nach Anspruch 1, **dadurch gekennzeichnet, dass** als Quaternierungsmittel der Formel $X\text{-}CH_2CN$ Chloracetonitril verwendet wird.

5. Verfahren nach Anspruch 1, **dadurch gekennzeichnet, dass** die Menge an Quaternierungsmittel der Formel $X\text{-}CH_2CN$ 1,8 bis 3,0 bezogen auf das tertiäre Diamin), beträgt.

6. Verfahren nach Anspruch 1, **dadurch gekennzeichnet, dass** die Menge an Quaternierungsmittel der Formel $X\text{-}CH_2CN$ 2,0 bis 2,5 bezogen auf das tertiäre Diamin), beträgt.

7. Verfahren nach Anspruch 1, **dadurch gekennzeichnet, dass** die Menge an Quaternierungsmittel der Formel $X\text{-}CH_2CN$ 2,0 bis 2,3 bezogen auf das tertiäre Diamin, beträgt.

8. Verfahren nach Anspruch 1, **dadurch gekennzeichnet, dass** man mit Essigsäureethylester, Essigsäure-n-propylester, Essigsäure-i-propylester, Essigsäure-n-butylester, Essigsäure-i-butylester und deren Gemischen, Dimethylsulfoxid, N-Methylpyrrolidon, 1,3-Dimethylimidazolidin-2-on als Lösungsmittel arbeitet.

9. Verfahren nach Anspruch 1, **dadurch gekennzeichnet, dass** man in Gegenwart von Essigsäureethylester, Dimethylformamid und Dimethylacetamid arbeitet.

10. Verfahren nach Anspruch 1, **dadurch gekennzeichnet, dass** man mit Dimethylacetamid als Lösungsmittel arbeitet.

11. Verfahren nach Anspruch 1, **dadurch gekennzeichnet, dass** man bei 20 bis 120°C arbeitet.

12. Verfahren nach Anspruch 1, **dadurch gekennzeichnet, dass** man bei 30 bis 100°C arbeitet.

13. Verfahren nach Anspruch 1, **dadurch gekennzeichnet, dass** man bei 40 bis 80°C arbeitet.

14. Verfahren nach Anspruch 1, **dadurch gekennzeichnet, dass** die Reaktionsdauer 1 bis 10 Stunden beträgt.

15. Verfahren nach Anspruch 1, **dadurch gekennzeichnet, dass** die Reaktionsdauer 2 bis 9 Stunden beträgt.

16. Verfahren nach Anspruch 1, **dadurch gekennzeichnet, dass** die Reaktionsdauer 3 bis 8 Stunden beträgt.

## Claims

1. Method for producing bifunctional ammonium nitriles of the formula

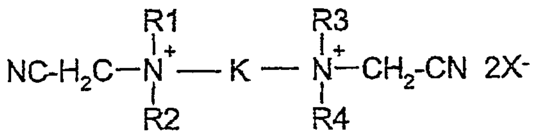

where K is a group of the formulae

$$-(CH_2)_n-,\ -(CH_2)_n-A-(CH_2)_n-,$$

A is oxygen or a group of the formula $N-R^5$
$R^1$, $R^2$, $R^3$ and $R^4$, in each case independently of one another, are a straight-chain or branched-chain $C_1$-to $C_{24}$-alkyl, alkenyl or alkyl ether group, preferably a $C_1$- to $C_{18}$-alkyl, alkenyl or alkyl ether group, or
$R^1$, $R^3$ and K, together with the two N atoms, are either a group of the formula

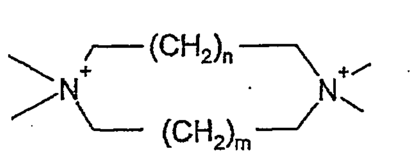

or $R^1$ , $R^2$ , $R^3$ , $R^4$ and K, together with the two N atoms, are a group of the formula

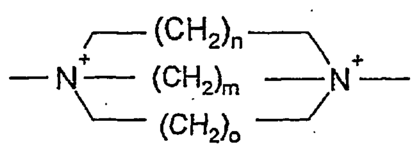

,

$R^5$ is hydrogen or a $C_1$- to $C_{24}$-alkyl , alkenyl or cycloalkyl group, preferably a $C_1$- to $C_{18}$-alkyl, alkenyl or cycloalkyl group,

$X^-$ is an anion, for example chloride, bromide, iodide, toluenesulfonate, benzenesulfonate, cumenesulfonate or mesitylsulfonate and

the variables m, n and o are integers from 1 to 16, by reaction of a tertiary diamine of the formula

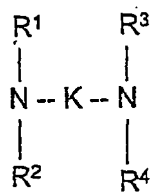

with a cyanomethylene-group-transferring quaternizing agent of the general formula

$X\text{-}CH_2CN$

**characterized in that** the reaction is carried out in a polar, aprotic solvent with a boiling point above 60°C, where A, $R^1$, $R^2$, $R^3$, $R^4$, $R^5$, X, m, n and o have the meanings given above.

**2.** Method according to Claim 1, **characterized in that** the quaternizing agent of the formula $X\text{-}CH_2CN$ used is haloacetonitriles and arylacetonitriles.

**3.** Method according to Claim 1, **characterized in that** the quaternizing agent of the formula $X\text{-}CH_2CN$ used is chloroacetonitrile, bromoacetonitrile, iodoacetonitrile, tosylacetonitrile and cumeneacetonitrile.

**4.** Method according to Claim 1, **characterized in that** the quaternizing agent of the formula $X\text{-}CH_2CN$ used is chloroacetonitrile.

**5.** Method according to Claim 1, **characterized in that** the amount of quaternizing agent of the formula $X\text{-}CH_2CN$ is 1.8 to 3.0, based on the tertiary diamine.

**6.** Method according to Claim 1, **characterized in that** the amount of quaternizing agent of the formula $X\text{-}CH_2CN$ is 2.0 to 2.5, based on the tertiary diamine.

**7.** Method according to Claim 1, **characterized in that** the amount of quaternizing agent of the formula $X\text{-}CH_2CN$ is 2.0 to 2.3, based on the tertiary diamine.

**8.** Method according to Claim 1, **characterized in that** the solvents used are ethyl acetate, n-propyl acetate, isopropyl acetate, n-butyl acetate, isobutyl acetate and mixtures thereof, dimethyl sulfoxide, N-methylpyrrolidone, 1,3-dimethylimidazolidin-2-one.

**9.** Method according to Claim 1, **characterized in that** it is carried out in the presence of ethyl acetate, dimethylformamide and dimethylacetamide.

**10.** Method according to Claim 1, **characterized in that** the solvent used is dimethylacetamide.

**11.** Method according to Claim 1, **characterized in that** it is carried out at 20 to 120°C.

**12.** Method according to Claim 1, **characterized in that** it is carried out at 30 to 100°C.

**13.** Method according to Claim 1, **characterized in that** it is carried out at 40 to 80°C.

**14.** Method according to Claim 1, **characterized in that** the reaction time is 1 to 10 hours.

**15.** Method according to Claim 1, **characterized in that** the reaction time is 2 to 9 hours.

**16.** Method according to Claim 1, **characterized in that** the reaction time is 3 to 8 hours.

## Revendications

**1.** Procédé de préparation de nitriles d'ammonium bi-fonctionnels de formule

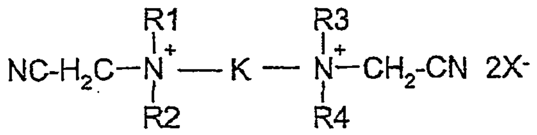

dans laquelle K représente un groupe de formules

$-(CH_2)_n-$, $-(CH_2)_n-A-(CH_2)_n-$,

A représente un atome d'oxygène ou un groupe de formule $N-R^5$,
$R^1$, $R^2$, $R^3$ et $R^4$ représentent chacun, indépendamment les uns des autres, un groupe alkyle, un groupe alcényle ou un groupe étheralkylique en $C_1$ à $C_{24}$, linéaire ou ramifié, de préférence un groupe alkyle, un groupe alcényle ou un groupe étheralkylique en $C_1$ à $C_{18}$, ou
$R^1$, $R^3$ et K, conjointement avec les deux atomes d'azote, représentent un groupe de formule

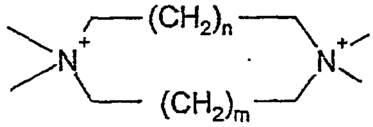

ou $R^1$, $R^2$ $R^3$, $R^4$ and K, conjointement avec les deux atomes d'azote, représentent un groupe de formule

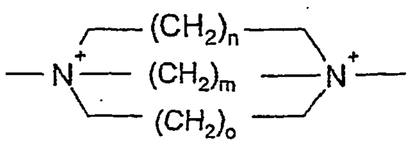

,

$R^5$ représente un atome d'hydrogène ou un groupe alkyle, un groupe alcényle ou un groupe cycloalkyle en $C_1$ à $C_{24}$, de préférence un groupe alkyle, un groupe alcényle ou un groupe cycloalkyle en $C_1$ à $C_{18}$,
$X^-$ représente un anion, par exemple, un chlorure, un bromure, un iodure, un toluène-sulfonate, un benzène-sulfonate, un cumène-sulfonate ou un mésityl-sulfonate, et
les variables m, n et o représentent des entiers de 1 à 16,
par la réaction d'une diamine tertiaire de formule

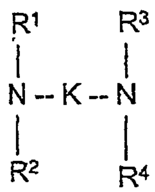

avec un agent de quaternisation transférant des groupes cyanométhylène de formule générale

$X-CH_2CN$

**caractérisé en ce que** la réaction est effectuée dans un solvant aprotique polaire présentant un point d'ébullition supérieur à 60°C,

où A, $R^1$, $R^2$, $R^3$, $R^4$, $R^5$, X, m, n et o présentent les significations indiquées ci-dessus.

2. Procédé selon la revendication 1, **caractérisé en ce que** l'on utilise des halogénoacétonitriles et des arylacétonitriles en tant qu'agents de quaternisation de formule $X\text{-}CH_2CN$.

3. Procédé selon la revendication 1, **caractérisé en ce que** l'on utilise le chloroacétonitrile, le bromoacétonitrile, l'iodoacétonitrile, le tosylacétonitrile et le cumène-acétonitrile en tant qu'agent de quaternisation de $X\text{-}CH_2CN$.

4. Procédé selon la revendication 1, **caractérisé en ce que** l'on utilise le chloroacétonitrile en tant qu'agent de quaternisation de formule $X\text{-}CH_2CN$.

5. Procédé selon la revendication 1, **caractérisé en ce que** la quantité d'agent de quaternisation de formule $X\text{-}CH_2CN$ est de 1,8 à 3,0, par rapport à la diamine tertiaire.

6. Procédé selon la revendication 1, **caractérisé en ce que** la quantité d'agent de quaternisation de formule $X\text{-}CH_2CN$ est de 2,0 à 2,5, par rapport à la diamine tertiaire.

7. Procédé selon la revendication 1, **caractérisé en ce que** la quantité d'agent de quaternisation de formule $X\text{-}CH_2CN$ est de 2,0 à 2,3, par rapport à la diamine tertiaire.

8. Procédé selon la revendication 1, **caractérisé en ce que** l'on travaille avec de l'acétate d'éthyle, de l'acétate de n-propyle, de l'acétate d'isopropyle, de l'acétate de n-butyle, de l'acétate d'isobutyle et leurs mélanges, du diméthylsulfoxyde, de la N-méthylpyrrolidone, de la 1,3-diméthylimidazolidin-2-one, en tant que solvant.

9. Procédé selon la revendication 1, **caractérisé en ce que** l'on travaille en présence d'acétate d'éthyle, de diméthylformamide et de diméthylacétamide.

10. Procédé selon la revendication 1, **caractérisé en ce que** l'on travaille avec du diméthylacétamide en tant que solvant.

11. Procédé selon la revendication 1, **caractérisé en ce que** l'on travaille entre 20 et 120°C.

12. Procédé selon la revendication 1, **caractérisé en ce que** l'on travaille entre 30 et 100°C.

13. Procédé selon la revendication 1, **caractérisé en ce que** l'on travaille entre 40 et 80°C.

14. Procédé selon la revendication 1, **caractérisé en ce que** la durée de la réaction est de 1 à 10 heures.

15. Procédé selon la revendication 1, **caractérisé en ce que** la durée de la réaction est de 2 à 9 heures.

16. Procédé selon la revendication 1, **caractérisé en ce que** la durée de la réaction est de 3 à 8 heures.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 303520 A
- EP 458396 A
- EP 464880 A
- WO 2003078561 A